Europäisches Patentamt

**European Patent Office**

Office européen des brevets

(19)

(11) Publication number: **0 168 007**

A2

(12)

# EUROPEAN PATENT APPLICATION

(21) Application number: **85108402.0**

(22) Date of filing: **06.07.85**

(51) Int. Cl.⁴: **A 61 B 5/10**
**G 07 C 9/00**

(30) Priority: **12.07.84 US 630012**

(43) Date of publication of application:
**15.01.86 Bulletin 86/3**

(84) Designated Contracting States:
**AT BE CH DE FR GB IT LI NL SE**

(71) Applicant: **FINGERMATRIX INC.**
**30 Virginia Road**
**North White Plains New York 10603(US)**

(72) Inventor: **Schiller, Michael**
**4465 Douglas Avenue**
**Riverdale, N.Y. 10471(US)**

(74) Representative: **Berkenfeld, Helmut, Dipl.-Ing.**
**An der Schanz 2**
**D-5000 Köln 60(DE)**

(54) Direct finger reading.

(57) A fingerprint image is derived from the direct optical reading of a finger by an optical and electronic scan of the finger with a beam of coherent, collimated light. The beam is shaped in the form of a slit of light which extends the length of the finger zone to be interrogated. That slit light beam is scanned across the finger in a direction perpendicular to the axis of that slit. The rays which constitute the interrogating light in the slit define an incident light beam in the form of a plane which is substantially perpendicular to the surface of the finger being interrogated. The light beam and the rest of the optical system is rotated about an axis that is substantially coincident with the axis of the finger so that this perpendicular relationship between the plane of the interrogating light beam and the surface of the finger is maintained throughout the scan.

EP 0 168 007 A2

## DIRECT FINGER READING
### Background of the Invention

This invention relates generally to an optical finger image generating apparatus and, more particularly to an optical finger image apparatus which generates an image from a scan of a finger object without a platen.

Fingerprint apparatus capable of generating a fingerprint image which can be encoded into machine readable signals are known in the art. Examples, of such apparatus are described in issued U.S. Patent No. 4,322,163. This apparatus generates a fingerprint image using substantially collimated light as an interrogating light beam which is displaced across a platen upon which a finger is supported. The finger on the platen modulates the interrogating light beam to provide a reflected light beam which has fingerprint information. Optical scanning means are used which cause the interrogating light beam to scan the fingerprint object carried by the finger on the platen. The modulated light beam is imaged onto an array of photo-electric transducers to produce a series of output signals indicative of the modulated information. The output of the array is serially interrogated at successive scan positions to provide a set of signals containing fingerprint information.

In the art of optical finger image processing, it is desirable to keep the system as inexpensive as possible and the size of the system as small as possible while ensuring that the reliability of the system is maintained. In order to get a useful fingerprint image the background signal must be kept as low and as steady as possible. Further, there must be high resolution of the fingerprint object. Prior art apparatus while providing a reliable system with high resolution and a controlled background signal uses platens upon which the finger being scanned is supported. Although the platens help to control the background signal and aid in resolution they also create problems. With use, deposits of oil, grease and dirt build up on the platen which affect the fingerprint image. Further, the pressure of a finger against the platen distorts the fingerprint object and the distortion is somewhat different each time the finger is applied to the platen.

These distortions arise out of at least four types of situations. There is a tendency for the ridges to close down on the valleys in the higher frequency portions of a finger when the finger is pressed against a platen. The positioning of a finger on a platen requires movement of the finger along the platen to a predetermined position and this generates a slip-stick distortion due to the friction of the finger on the platen surface. A third type of distortion is latent image distortion which arises from the latent images left by previous applications of a finger to a platen. A fourth type of distortion occurs because of the interaction of the finger oil with the platen. This last distortion is particularly severe with fingers which generate a great deal of finger oil. All these distortion situations tend to be non-repeatable so that the finger image varies as between applications of the same finger to a platen. This complicates analysis and requires relatively expensive processing to compensate for these distortions.

U.S. Patent No. 3,614,747 to Charles Sadowsky discloses a fingerprint apparatus which uses regular light to scan a portion of a finger through a slit. The Sadowsky apparatus uses and must use a point illumination to keep the background light level (that is, the base line in the electrical signal) as low as possible. Sadowsky employs a shadowing technique to generate differential illumination of ridges and valleys by scanning an interrogating point of light along a single slit aperture.

It is an object of the present invention to provide an optical finger image processing apparatus which produces high resolution fingerprint images without using a platen.

Another object of the present invention is the provision of such an apparatus which avoids swamping out the image signal with the base line signal.

It is a further object of the present invention to provide such an apparatus which is relatively inexpensive, reliable and provides a repeatable image.

Still a further object of the present invention is the provision of such an apparatus which is relatively small in size.

## Brief Description Of The Drawings

FIG. 1 is an optical and mechanical schematic of the device of this invention showing a finger in position being interrogated by a light beam 22.

FIG. 2 is a schematic idealized representation of the typical relationship between the ridge and valley zones of an undistorted finger.

## Brief Description

In brief, the device of this invention provides for the direct reading of a finger object with a coherent, collimated light beam in which the angle of incidence of the light beam on the finger surface being interrogated is sufficiently perpendicular so that the modulation in the reflected light beam is due to constructive and destructive reinforcement of the coherent light rather than due to shadowing.

In one embodiment, the coherent, collimated interrogating light beam is shaped in a flat planar like beam that impinges on the finger as a slit of light. The plane of the interrogating light beam and the plane of the reflected modulated light beam are both essentially coplanar and are substantially perpendicular to the surface of the finger along the line of interrogation. The line of interrogation is parallel to the axis of the finger being interrogated. This planar interrogating light beam is scanned across the finger in a direction perpendicular to the plane of the light beam. The optical scan involves rotation of the light beam so as to maintain the substantially perpendicular relationship between the surface of the finger along the line of illumination and the plane of the interrogating light beam.

The substantial perpendicularity of the plane of the interrogating and reflected light beams to the surface of the finger avoids the geometric distortion that would be associated with the reading by a shadowing technique. The shadowing technique, because it requires a non-perpendicular beam, causes geometric distortion. The use of coherent light provides a mode of illumination that enhances the distinction between the ridge zones and valley zones in the image plane. Two spaced apart imaging lenses are positioned in the path of the reflected modulated light beam to provide an image at an array of photoelectric transducers. The two spaced apart imaging lenses augment focal depth so that sharply focused fingerprint image can be produced at the array of photoelectric transducers.

A laser provides the coherent, substantially collimated light which is shaped into a line of light by cylindrical lenses to form the interrogating light beam. The interrogating light beam is scanned across the finger to interrogate that portion of the finger that extends beyond a support and to produce the reflected light beam having fingerprint information.

The line of incidence of the interrogating light beam on the finger is parallel to the axis of the finger. The optical system, inluding the laser, is mounted on a platform which rotates about the axis of the finger so as to provide a scan which can run from nail to nail. Because of the rotation of the optical system around the axis of the finger, the interrogating light rays travel along a plane which remains substantially normal to the finger surface. Within that plane, the collimated light is incident on the finger surface at an angle that is off-normal by about four degrees (4°). Thus, the reflected modulated light beam is also off-normal by an angle of about four degrees and will travel along a line through the focusing lenses to the transducer array.

Thus, it may be seen that the plane of light that defines the interrogatory line is perpendicular to the surface of the finger. This avoids the geometric distortion that would accompany a shadowing illumination technique. Then by using coherent light, the interaction of that illumination with the finger surface results in the differental modulation of ridge zones and valley zones; a differential modulation that is believed to be due to destruction phase interference in the valley zones and constructive phase interference in the ridge zones.

## Description of the Preferred Embodiment

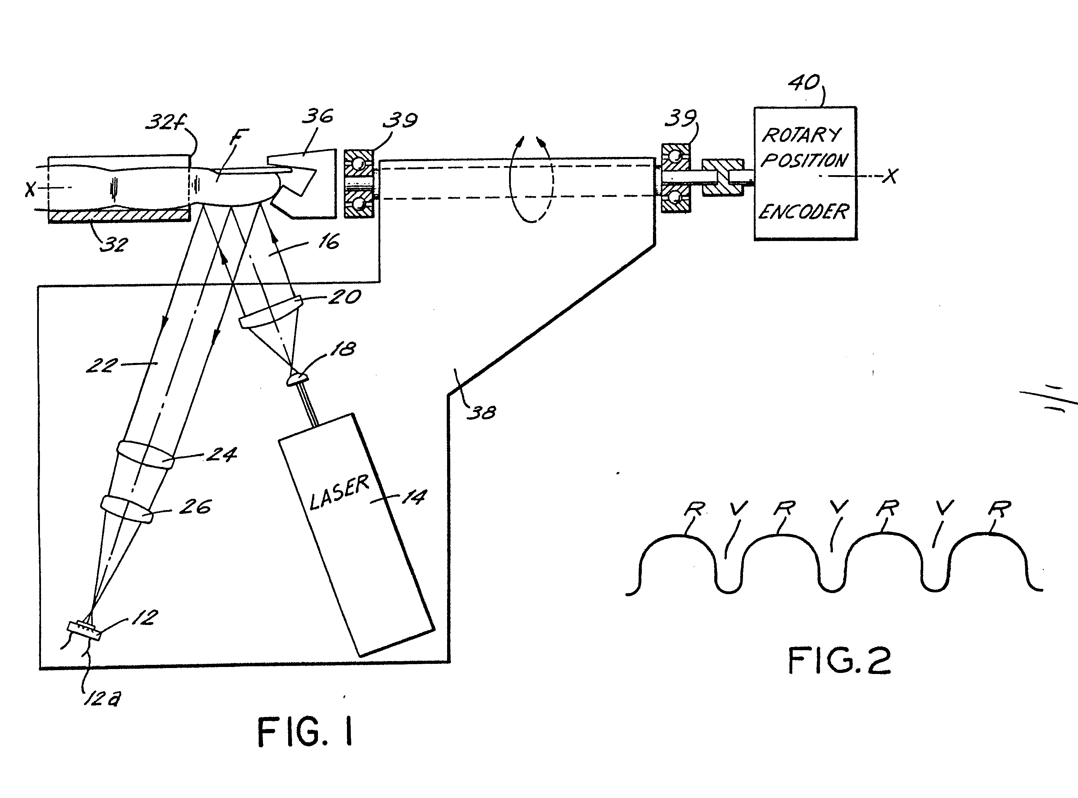

Referring now to the drawings, FIG. 1 illustrates the improved optical finger image processing apparatus of the present invention. The apparatus includes a linear photodiode array 12 which is conventional in construction and may, for example, comprise charge coupled diode array Model No. CCD 133 manufactured by the Fairchild Semi-Conductor Divison of Fairchild Camera & Instrument Co., of Mountainview, California. This particular array comprises 1,024 photodiodes that extend in a longitudinal direction. The diodes are aligned in contact with one another and have a center to center spacing of about 0.014mm (o.55 mils). Accordingly, the shape of the light receiving opening of the array is in the form of a slit wherein the long dimension of the slit corresponds to the longitudinal direction of the array.

A laser 14 is used as a source of an interrogating light beam 16. Laser 14 provides a beam of coherent substantially collimated light. As used herein collimated light refers to a light beam in which the individual rays do not scatter. It is not essential that they never cross over one another. Thus, a collimated light beam may be diverging, parallel or converging.

Cylindrical condensing lenses 18 and 20 are positioned in the path of interrogating light beam 16 to shape the light into a line and to concentrate the light so that adequate light intensity is provided.

Interrogating light beam 16 is modulated to contain fingerprint information, as explained hereinafter. The modulated light beam is referred to herein as reflected light beam 22. Reflected light beam 22 contains fingerprint information.

Two imaging lenses 24 and 26 are provided which project the fingerprint image from the reflected light beam 22 onto CCD array 12. The fingerprint image projected onto array 12 contains light and dark spots indicative of fingerprint information. The relationship between imaging lenses 24 and finger F and between imaging lens 26 and array 12 is of importance to the practical implementation of the present invention and will be explained more fully hereinafter.

A rotatable support member 38 provides a mount for the laser 14, the beam forming lenses 18 and 20 and the imaging lenses 24 and 26 as well as the CCD array 12. This support 38 is rotatable about the axis X-X and in use is rotated in an arcuate fashion over an arc of between $90^{\circ}$ and $180^{\circ}$ so that the interrogating light beam 16 strikes the finger F with a substantially constant angle of incidence.  The axis X-X is substantially the axis of the finger F being interrogated.  The support element 38 is mounted for rotational movement on bearings 39.

Coupled to the shaft on which the support element rotates is a rotary position encoder 40.  The encoder 40 is conventional and produces a signal each time the support 38 rotates an incremental distance.  In the array 12, each of the diodes are about 0.02 mm (about 1 mil) on a side.  The encoder 40 is callibrated to produce a synchronizing signal each time the support rotates through an arc which displaces the image at the array about 0.02 mm.  Simultaneous with the initiation of the scan, the encoder 40 produces a synchronizing signal which is applied to an electronic scanning circuit which is coupled to the CCD array output on the leads 12a.  The electronic scanning circuit is conventional in construction and is adapted to serially interrogate each of the CCD elements that comprise the array 12 in response to the syncronizing signal.  Thus, the optical scan which is along one axis (perpendicular to the axis X-X) is electronically scanned along the other axis (the line of the array 12) in a periodic fashion that produces a series of signals that constitute the picture elements or pixels of the image being generated.

The interrogating light beam 16 is shaped by the lenses 18 and 20 to provide a long, narrow, planar-like interrogating light beam. The length of the light beam as incident on the Finger is about 2.5cm in one embodiment substantially parallel to the axis X-X. The width of the light beam is about 0.1mm to 0.12mm at the Finger F and is along a direction substantially orthogonal to the direction of the axis X-X.

Thus, the light rays are concentrated to provide a narrow slit illumination. The light rays that are within this interrogating slit define a plane that intersects the axis X-X and is substantially perpendicular to the surface of the finger F at the line of interrogation. Rotation of the optical system about the axis X-X maintains this perpendicular relation throughout the scan. Because the plane of the incident light beam 16 is perpendicular to the surface of the finger F, the plane of the reflected light beam 22 is also perpendicular to the surface of the finger F. Indeed those two planes are co-planar.

However, within the plane of the interrogating beam 16, the incident rays are at a small angle to the finger surface line being interrogated. For purposes of illustration, FIG. 1 exaggerates the angle of incidence

0168007

of the interrogating rays. In one embodiment the angle is only four degrees (4°) inthe plane of FIG. 1.

Apparatus 10, unlike prior art aparatus does not use a platen. Rather, the finger F is held on a finger support 32 and positioned by fingertip positioning element 36. The two supports 32 and 36 are spaced from one another. Thus, a portion of finger F extends beyond the front edge 32f of support 32. Although it is preferable to use support 32, it is possible to practice the present invention without such a support if the finger to be scanned is held still in proper position in the path of interrogating beam 16.

The finger F on the support 32 is positioned generally at the front focal plane of the first imaging lens 24. Reflected light rays form the finger pass through lens 24 and are substantially collimated by lens 24. These substantially collimated reflected light rays then pass throgh imaging lens 26. Due to the collimated nature of these reflected rays, lens 26 processes them as if coming from an infinite distance. In this way depth of field is augmented within the confines of a relatively small apparatus 10 to thereby produce a focused fingerprint image at array 12. The array 12 is positioned at the back focal plane of the lens 26.

The ratio of the distance between finger F and lens 24 and the distance betwen array 12 and lens 26 determines the minification value of system 10. For example, in one embodiment of the present invention, the distance between the finger F and lens 24 is 20cm while the distance between the array 12 and lens 26 is 10cm thereby providing a minification value of two.

That embodiment provides a 0.028mm (about 1.1 mil) resolution of the finger object. The array 12 has a 0.014mm (0.55 mil) center to center distance between cells. The average ridge to ridge spacing of a finger is about 0.4mm (15 mils).

The use of coherent, substantially collimated light and the cylindrical shaping lenses are part of the reason why the background signal can be kept reasonably low.

An embodiment has been tested. It produces an image which has a distinct bright line along the crest of the ridge of the fingerprint and a substantially low level or dark zone between crests. The bright line constitutes perhaps ten to fifteen percent of the ridge to ridge distance and the dark zone constitute the other 85 to 90 percent of that distance. The bright crest line appears to be a result of reinforcement due to constructive interference of the reflected modulated light beam. The dark zone appears to represent a destructive interference of the reflected light. The background slowly varying DC component is sufficiently low relative to the amplitude of the crest line so that it can readily be subtracted out leaving a meaningful and useable image. In addition, the image comes through in substantially clear fashion over any background noise.

What has been found is that this result can be achieved only if coherent light is used. The precise mechanism by which the coherent light provides reinforcement along the crests of the ridges and cancellation over the rest of the image is not completely understood. The test embodiment has been checked by being illuminated with a light source which is incoherent, collimated and monochromatic. But that source fails to produce a meaningful, useable image comparable to that produced bythe coherent interrogating light beam. The system has also been checked with an optical inspection lamp which provides an interrogating beam that is incoherent, highly collimated but that is not monochromatic. The result is also useless. The conclusion reached is that coherency is an essential feature. Accordingly, it is concluded that the reinformcement along the ridge crests is due to constructive electrical phase interference and the substantial absence of light over the rest of the image is due to destructive electrical phase interference.

The constructive reinforcement that occurs in the light at the crest of the ridges is due to the varying nature of the object involved. FIG. 2 shows, in idealized form, the relationship of ridges to valleys as seen along a plane taken normal to the finger surface. The ridges R are substantially thicker than the valleys V. The ration between R and V may be four to one or five to one. When the finger object is applied to a platen, the center portion of the ridges are flattened out against the glass platen. The rest of the ridges and the valley zones are spaced back from the surface of the glass platen. Since the image reflected is that at the back surface of the glass platen, the result is an image that has ridge and valley zones which are much more similar in thickness to one another than exists in the actual object as illustrated in FIG. 2. In certain areas of the finger of some individuals, where the pressure of the finger agaist the platen is great enough, the ridges flatten out sufficiently to close down on the valleys. But normally a good part of the ridges do not even contact the platen surface.

As a practical matter, because the typical valley width is such a small fraction of the ridge to ridge spacing, it becomes necessary that the resolution of the system be at least as fine as about 0.04 mm (1.5 mils) per pixel. This will make sure that the valley width of the population of fingers will be appropriately imaged.

However, in connection with the direct platen-less finger reading of this invention, it may be seen tht the variation of optical distance along the ridge zone, is fairly slight relative to the variation in optical distance throughout the valley zone. It is believed that this is at the heart of why, with coherent light, constructive and destructive reinforcement is observed. Any small optical distance variation along at least a portion of the ridge zone is not sufficient to bring the reflected light out of phase sufficient to cause cancellation. It is believed that what happens is that points along the ridge surface which are close to one another but which have slightly different focal distances can and do end up as the same point in the image plane. As a consequence, there is constructive reinforcement. This provides substantially enhanced illumination along the center of the crest of the ridge zone in the image plane. Because the ridge surface does indeed have certain irregularities and optical distance variations, the reinforcement may not be uniform throughout the ridge surface and may not even be uniform throughout the crest of the ridge surface. However, it is sufficiently uniform to provide net reinforcement within each pixel along the crest of the resultant image at the resolution involved, which is a resolution of 0.04mm (1.5 mils) per pixel.

By contrast, the destructive interference which occurs in the light reflected out of the valley areas is a result of the fact that the more substantial and more varying differences in the focal lengths of various points in the valley zones results in electrical phase cancellation and thus a dark zone at the image plane.

Somewhat related to the above is the depth of focus consideration. It is important that the depth of focus be substantial because of the curvature of the finger object. That is, even the ridge crests shown in the image plane are not themselves in the same object plane. By providing a large depth of focus (and ideally an infinite depth of focus), the difference in optical distance along the finger surface makes little or no distinction as far as establishing an image at the CCD array. The large depth of focus means that if there were a sensible image from the areas between the ridge crests, it would be produced at the CCD array. The fact that there is no image in the zones between the crests of the ridges is a strong indication that cancellation of the reflected illumination from such zones on the finger object occurs.

Presumably any interogating light beam would provide a reflected light beam in which finger image modulation would exist to some extent. However, that image information would be so swamped out by the background illumination which results from the diffusing and varied nature of the finger object itself that there is no practical way to extract such information from the large and varying base line or pedestal within which it is effectively buried.

The geometric relationships discussed herein have assumed that the finger surface being scanned can be treated as a circular cylinder around the axis X-X. Althought the actual surface differs from that ideal concept, it is close enough so that the system as described operates in a practical embodiment to provide a practical result. Any deviations from that ideal still provides results which have fewer distortions or deviations than occur with a system employing a platen.

Thus, it may be seen that the configuration of the optical system of this invention provides for the direct illumination of a finger object without the geometric distortion that would occur if the entire two-dimensional finger area were to be illuminated by a shadowing technique. By maintaining the plane of the interrogating light beam substantially perpendicular to the surface of the finger along the active line of interrogation during the entire scan of the finger, the geometric distortion that would otherwise occur is avoided. The angle of incidence of the light rays within the incident plane are off-normal by a few degrees. But as long as that off-normal angle is maintained only along a single axis, the geometric distortion is avoided whether or not there is some degree of shadowing. If there is some degree of shadowing along an axis that is parallel to the axis of the finger, then there must be no shadowing or off-normal incidence along a line orthogonal to the axis of the finger.

1. Method of direct reading of a finger object, characterized by the steps of illuminating the finger object (F) with a substantially coherent, substantially collimated light beam (16) having an interrogating zone in the form of a slit, the light rays in the interrogating zone defining an interrogating light beam in the form of a plane, said plane being substantially perpendicular to the surface of the finger (F) being interrogated, and scanning the interrogating light rays that define said plane across said finger (F).

2. Method according to claim 1, characterized in that said plane is substantially parallel to a predetermined axis, said predetermined axis being approximately coincident with the axis of the finger (F) to be interrogated.

3. Method according to claim 2, characterized in that said plane intersects said predetermined axis.

4. Method according to claim 2, characterized in that said step of scanning is along a direction substantially perpendicular to said plane.

5. Method according to claim 1, characterized in that said step of illuminating and scanning provides a reflected light beam (22) having a modulated zone, the light rays in said modulated zone defining a light beam in the form of a second plane, said second plane being substantially perpendicular to the surface of the finger (F) being interrogated and being substantially co-planar with said plane of said interrogating light beam (16).

6. Method according to claim 5, characterized by the step of focusing the image carried by a reflected modulated light beam (22) onto a linear array (12) of photoelectric transducers downstream from the finger (F) being
interrogated.

7. Method according to claim 5, characterized in that said
   step of focusing provides a resolution of said transducers at least as fine as 0.04 mm of finger surface
   per pixel.

8. Method according to claim 1, characterized by the step
   of supporting said finger (F) in a substantially predetermined position and orientation with a support (38)
   that leaves a predetermined portion of said finger (F)
   uncovered to permit the direct interrogation of said
   portion by said interrogating light beam (16).

9. Method according to claim 1, characterized in that the
   interrogating light rays are incident on the finger surface being interrogated at a predetermined off-normal
   angle within said normal plane.

10. Optical finger image processing apparatus for carrying
    out the method according to claims 1 to 9, characterized by

          finger support means (38) adapted to support a
    finger (F) thereon such that a forward portion of any
    finger supported thereon will extend beyond a forward
    edge of said support means (38),

          means (14) to generate a coherent, substantially collimated light beam (16) positioned such that it
    can interrogate a portion of a finger (F) extending beyond said edge of said support means (38) to provide a
    reflected light beam (22) containing fingerprint information, and

          photo-electric transducer means (12) optically
    downstream from said finger support means (38) and positioned to receive the fingerprint image carried by
    said modulated light beam (22),

said interrogating light beam (16) having an interrogating zone in the form of a slit, light rays in said interrogating zone defining the interrogating portion of said light beam (16) in the form of a plane, said plane being substantially perpendicular to the surface of the finger (F) being interrogated,

said reflected light beam (22) including a modulated zone representing the reflection from said interrogating beam (16), said modulated zone being in the form of a plane, the plane of said modulated zone and the plane of said interrogating zone being substantially coplanar.

11. Apparatus according to claim 10, characterized in that the image provided at said transducer means (12) has a resolution at least as fine as 0.04 mm of finger surface per pixel.

12. Apparatus according to claim 10, characterized by an imaging lens means (24, 26) positioned in said reflected light beam (22), said imaging lens means (24, 26) capable of augmenting depth of focus to thereby produce a focused fingerprint image at said array (12).

13. Apparatus according to claim 10, characterized by cylindrical lens means (18, 20) positioned in the path of said coherent substantially collimated light beam (16) to shape said light beam (16) into said plane format.

14. Apparatus according to claim 12, characterized by cylindrical lens means (18, 20) positioned in the path of said coherent substantially collimated light beam (16) to shape said light beam (16) into said plane format.

15. Apparatus according to claim 12, characterized in that the image provided at said transducer means (12) has a resolution at least as fine as 0.04 mm of finger surface per pixel.

16. Apparatus according to claim 13, characterized in that the image provided at said transducer means (12) has a resolution at least as fine as 0.04 mm of finger surface per pixel.

17. Apparatus according to claim 14, characterized in that the image provided at said transducer means (12) has a resolution at least as fine as 0.04 mm of finger surface per pixel.

18. Apparatus according to claim 12, characterized in that said imaging lens means (24, 26) comprises first and second imaging lenses positioned in the path of said reflected light beam (22), said portion of said finger (F) extending beyond said edge of said support (38) being positioned generally at the front focal plane of said first imaging lens (24), said transducer means (12) being positioned at the back focal plane of said second imaging lens (26).

19. Apparatus according to claim 10, characterized by a fingertip positioner (36) spaced a predetermined distance from said edge of said finger support (38) to aid in supporting said finger (F).

0168007

FIG.1

FIG.2